## Europäisches Patentamt

## European Patent Office

## Office européen des brevets

(19)

(11) Publication number: **0 082 222**
**B1**

(12)

# EUROPEAN PATENT SPECIFICATION

(45) Date of publication of patent specification: **11.03.87**

(21) Application number: **81305952.4**

(22) Date of filing: **18.12.81**

(51) Int. Cl.⁴: **B 01 J 23/74,** B 01 J 35/10,
C 07 C 1/00

(54) Catalytic process for converting carbon monoxide to a high energy gas.

(43) Date of publication of application:
**29.06.83 Bulletin 83/26**

(45) Publication of the grant of the patent:
**11.03.87 Bulletin 87/11**

(84) Designated Contracting States:
**BE CH DE FR GB IT LI LU NL SE**

(56) References cited:
**EP-A-0 004 456
DE-A-2 715 623
DE-B-2 653 986
GB-A-2 002 726
US-A-3 038 865
US-A-3 933 883
US-A-4 016 108**

(73) Proprietor: **THE STANDARD OIL COMPANY
Midland Building
Cleveland, Ohio 44115 (US)**

(72) Inventor: **Velenyi, Louis Joseph
1266 Roland Road
Lyndhurst Ohio 44124 (US)**
Inventor: **Hardman, Harley Foch
4989 Delevan Drive
Lyndhurst Ohio 44124 (US)**

(74) Representative: **Smith, Sydney et al
Elkington and Fife High Holborn House 52/54
High Holborn
London WC1V 6SH (GB)**

Courier Press, Leamington Spa, England.

# 0 082 222

**Description**

This invention relates to a process of converting carbon monoxide to a high energy gas utilizing a particular catalytic material.

In EP—A—0,004,456 there is disclosed a process for methanation of carbon monoxide without prior separation of inert gas. The process involves passing a carbon monoxide-containing gas stream over a catalyst capable of catalysing disproportionation of carbon monoxide to carbon dioxide and an active surface carbon which is deposited on the catalyst. Steam or a steam-containing gas or hydrogen or hydrogen-containing gas stream is then passed over the active surface carbon to convert it to methane and carbon dioxide. In this manner relatively pure methane can be obtained from carbon monoxide containing gas streams without the necessity of prior separation of inert gases.

The catalyst employed preferably has a surface area of at least 10 $m^2/g$ in particular at least 25 $m^2/g$. No reference is made to the porosity of the catalyst. The catalyst can include as catalytic material transition metals of which reference is made to nickel, cobalt, iron, ruthenium, rhenium and alloys thereof.

Belgian Patent No. 869,185, issued January 22, 1979 describes a novel catalytic material comprising carbon fibers containing small nodules of iron or another Group VIII metal. This material is formed by contacting the Group VIII metal with a mixture of carbon monoxide and hydrogen at elevated temperature to cause deposition of carbon through the disproportionation of carbon monoxide. When the material so formed is contacted with hydrogen at elevated temperature, the carbon in the material reacts with the hydrogen to form methane. Repeated cycling between carbon deposition and methanation causes the catalytic material to exhibit high activity, i.e. high carbon deposition rates and high methanation rates.

In addition to very high catalytic activity, this material exhibits other unusual properties. For example, the material undergoes drastic changes in volume between the carbon deposition and methanation steps. Thus, it has been found that the volume of the material at the end of a carbon deposition step can be as much as 20 times as great as the volume at the end of a methanation step. Also, it has been found that repeated carbon deposition/methanation cycling causes gradual pulverization of the Group VIII metal on which the carbon fibers are grown. Also, the carbon fibers, especially when grown from pulverized metal produced by repeated cycling can exhibit extremely great expansive forces. For example, in one instance a stainless steel laboratory reactor was actually split open by the carbon fibers grown therein when the volume of the carbon fibers exceeded the internal volume of the reactor.

Because of the high catalytic activity of this material, it has been proposed to use this material for the production of high energy gas from coal. This would be accomplished by gasification of the coal to produce producer gas and reacting the carbon monoxide in the producer gas in accordance with the above technique to produce methane. Unfortunately, the unusual properties of this material make it difficult or impossible to handle in conventional gas/solid contacting apparatus such as fixed or fluid-bed reactors wherein a bed of the solid is continuously contacted with a gas flowing therethrough.

For example, in fixed bed operation, destruction of the reactor by growing too much carbon fiber material is always a possibility. Also, the powdery residue produced by repeated carbon deposition/methanation cycles tends to collect together and on subsequent carbon deposition steps the carbon/metal mass produced agglomerates and plugs the reactor inlets and outlets.

In fluid-bed operation, it is necessary that the solid being contacted has a relatively specific particle size distribution and density. The vast changes in volume as well as density of the material and the fragile nature of the carbon fibers makes the material totally unsuitable for fluid-bed operation.

In US—A—3,933,883 there is described a methanation reaction, that is to say the conversion of carbon monoxide and hydrogen to methane and by-product water, in which there is used as a catalyst a catalyst consisting of nickel oxide and cobalt oxide in a nickel/cobalt ratio of 1 to 1.5 supported on a high purity alumina support. There is disclosure of catalysts in which the surface area of the catalyst support is 155 $m^2/g$ or 299 $m^2/g$ and the water pore volumes is 0.58 $cm^3/g$ and 0.77 $cm^3/g$ respectively.

In US—A—3,038,865 there is disclosure of aluminium oxide reforming catalysts which consist of alumina and platinum. The surface area of various aluminas is given in the table in Example 2, as is the pore volume.

There is no disclosure in either of the above specifications of the use of the catalysts described therein in a two-step process for the conversion of carbon monoxide to methane.

We have found that a catalytic material which has physical characteristics similar to those known from the art just referred to is capable of catalyzing carbon deposition and methanation reactions with high catalytic activity and has a combination of physical properties making it amenable for use in a conventional fluid-bed reactor.

The present invention provides a fluid-bed process for converting carbon monoxide to a high energy gas comprising contacting a particulate, free-flowing, fluid-bed catalyst comprising a porous catalyst support having a surface area of greater than 1 to less than 350 $m^2/g$ and a pore volume of 0.4 to 3.0 ml/g and a Group VIII metal or mixture thereof in elemental form on the pore surface of said support, said Group VIII metal or mixture being present in an amount not exceeding that equivalent to at least five atomic layers with an upflowing gas stream containing carbon monoxide so as to fluidize said catalytic material and deposit carbon in the pores of said catalytic material and thereafter contacting said catalyst with a carbon

2

removal gas selected from hydrogen and steam so as to fluidize said catalytic material and remove carbon from said catalytic material.

The catalytic material used according to the invention is composed of a porous fluid-bed support material having a specific combination of properties which is impregnated with iron or other Group VIII metal so that the metal is present on the inside surfaces of the particulate support, that is on the pore surfaces.

Particles composed of a porous particulate material such as silica impregnated with iron are well known. In accordance with the invention, however, the nature of the pores must be precisely selected, that is the particles must exhibit a precise combination of properties due to the pores, in order that the catalytic material used according to the invention will exhibit the necessary properties. If the support material does not exhibit the desired combination of properties, the resultant material will be ineffective as a catalyst either because it cannot catalyze the carbon deposition and/or methanation steps with appropriate catalytic activity or it will be dimensionally unstable.

Support material

Porous, particulate, free-flowing catalyst support materials for use in making fluid-bed catalysts are well known. Examples of such materials are silica, alumina, silica/alumina, zirconia, titania, hafnia, silicon carbide, boron phosphate, diatomaceous earth, pumice and so forth.

In accordance with the invention, any such support material can be employed to make the catalytic material according to the invention provided that such support material has a surface area of greater than 1 to less than 350 m²/g, a pore volume of 0.4 to 3 ml/g and a particle size distribution and density appropriate for use as a fluid-bed material. Preferred are those supports having a surface area of 6 to 250 m²/g and pore volumes of 1 to 2 ml./g. Even more preferred are supports having a surface area of 30 to 80 m²/g and a pore volume of 1 to 2 ml./g.

Especially preferred support materials are those as described above which are further characterized in containing no measurable pores having pore diameters less than 50 Å (5 nm) preferably less than 80 Å (8 nm), more preferably less than 100 Å (10 nm) when measured by the mercury porosimeter techniques. In accordance with this technique, which is described in H. M. Rootare and C. F. Prenzlow, *Surface Area from Mercury Porousimeter Measurements,* Journal of Physical Chemistry, *71,* 2733 (1967), a mixture of mercury and the porous material to be tested is subjected to increasing pressure whereby mercury is forced into the pores of the material. A decrease in volume of the mixture corresponds to the amount of mercury taken up by the pores of the material. As the diameter of the pores decreases, greater pressure is needed to force mercury into the pores, and this relationship (pressure versus pore diameter) is known. Thus measurement of the pressure at any one point of time as the mixture is being subjected to increasing pressure indicates the diameter of the pores being filled with mercury at that point in time. In accordance with the preferred embodiment of the invention, porous support particles are used which show no measurable pores having a pore diameter of less than 50 Å (5 nm), preferably 80 Å (8 nm) and more preferably 100 Å (10 nm). This is reflected by the fact that once the pressure corresponding to 50 (or 80 or 100) Å (5 (or 8 or 10) nm) is reached, there is no more volume decrease and hence no more uptake of mercury by the pores of the support even if the pressure is further increased. This does not mean that the particles contain no pores having a pore diameter of 50 Å (5 nm) or less, but rather just that the mercury porousimeter technique is unable to mesure the presence of such pores.

Although not wishing to be bound to any theory, it is believed that the importance of the above surface area and pore volume restrictions relates to the elimination of extremely small pores from the support particles. This is reinforced by the further positive restriction that no measurable pores of the pore sizes indicated above be present. If carbon were allowed to grow in extremely small pores in the support particles, it would soon fill up these pores. Continued growing of the carbon would cause high stresses to be exerted on the particle skeleton which would ultimately lead to fracturing of the particles. Hence, by eliminating the very small pores, the tendency of the particles to break up because of the high compressive stresses developed when the carbon material outgrows its growing space is eliminated.

It is not known whether or not particulate support material having the combination of properties set forth above is readily available. These materials, however, can be easily produced by known processing techniques from support materials which are readily available.

For example, the preferred support material is porous particulate silica which is produced by subjecting a commercially available porous particulate silica to a hydrothermal technique to adjust the pores to the desired configuration. The commercially available silica used in this technique has the properties and characteristics as set forth in the following Table I.

# 0 082 222

### TABLE I

| Chemical compositions: | |
|---|---|
| Loss in ignition (1 hr. 1000°C) | 4.5 % wb* |
| $Al_2O_3$ | 0.3 % db** |
| $Na_2O$ | 0.07 % db |
| $SO_4$ | 0.08 % db |
| Fe | 0.03 % db |

| Physical properties: | |
|---|---|
| Apparent bulk density | 0.38 g/ml |
| Compacted bulk density | 0.47 g/ml |
| Surface area | 645 m²/g |
| Pore volume—$H_2O$ (6000°C) | 1.07 ml/g |

| Particle size distribution: (micromesh—square holes) | |
|---|---|
| — 149 micrometers | 98.5 % |
| — 105 micrometers | 95.5 % |
| — 90 micrometers | 90.5 % |
| — 45 micrometers | 32 % |
| — 30 micrometers | 10 % |
| — 20 micrometers | 1.5 % |

\* wb=percent wet basis
\*\* db=percent dry basis

This material is processed into the preferred support material by the following procedure. 40 gms. of the above material is placed in a 300 ml. glass autoclave liner with 100 ml. of dilute aqueous $K_2CO_3$ solution. The autoclave is then brought to 230°C and maintained at that temperature for 30 minutes. The pressure at this point is 400 psig (27,6 bars). At 30 minutes, the autoclave is rapidly cooled. The silica is then removed from the reactor, washed three times with 100 ml. aliquots of distilled water and then dried overnight in air at 110°C. The porous, particulate free-flowing silica product made in this manner exhibits a surface area of 53 m²/g. ($N_2$ adsorption technique), a pore volume (measured by the porousimeter technique) of 1.22 ml./g. and no measurable pores with pore diameter less than 150 Å (1,5 nm) when measured by the mercury porousimeter technique.

The above hydrothermal technique serves to reduce the surface area of the silica starting material, presumably through elimination of extremely small pores. Other techniques are known which will reduce surface area and otherwise adjust the pore structure of fluid-bed support materials, and any such technique can be employed in connection with suitable starting materials. For example, it is known that impregnation of a high surface area support material such as silica or alumina with ammonium heptamolybdate will, once the support is calcined in air or other atmospheres, significantly reduce the support surface area of the support material, this presumably being due to blockage of the small pores with molybdenum oxide. Impregnation of silica with phosphoric acid followed by calcination in air is also known to significantly reduce the surface area of porous silica. Any such techniques can be employed in accordance with the present invention to produce particulate support material of the appropriate properties.

As well known to those skilled in the art of fluid-bed reactions, a solid which is to be fluidized must have an appropriate particle size distribution and density. Since the vast majority of the Group VIII metal deposited on the support of the catalytic material will be incorporated into the support interior, the particle size distribution of the support will be similar or essentially the same as the particle size distribution of the catalytic material itself. The support, therefore, must also exhibit a particle size distribution appropriate for use as a fluid-bed material. Normally, this means that at least 80% of the particulate support will have a particle size distribution ranging between 5 to 300, preferably 10 to 200, more preferably 20 to 150 micrometers.

Group VIII metal

Any Group VIII metal or the mixture thereof can be employed as the coating or impregnant for the support pores surfaces of the catalytic material used according to the invention. Iron, cobalt and nickel are preferred, while iron and nickel are especially preferred.

The amount of Group VIII metal in the support can vary widely. For convenience, the amount of Group VIII element is normally set forth in terms of single layers of the Group VIII element based on the measured surface area of the support to be impregnated. In other words, based on the surface area of the support to be impregnated and the atomic radius of the Group VIII element to be deposited on the pore surfaces of the support, the total number of atoms and hence the total number of moles of Group VIII element needed to completely coat all the surfaces of the particles with a single layer of the Group VIII element one atom thick

4

can be calculated. Referring to the amount of Group VIII element impregnated into the support in terms of multiples or fractions of such layers has been found to be very convenient.

It is preferred that the amount of Group VIII element impregnated into the support particles be at least about one layer although the amount can be less. Metal loadings substantially less than one layer have been found to produce a catalyst of very poor activity. As the metal loading increases above one layer, the performance of the catalyst increases rapidly with catalysts having a metal loading corresponding to two layers exhibiting excellent activity. As the metal loading increases above two layers, no significant advantage is realized. Theoretically, there is no maximum to the amount of metal loading except that too much metal will fill the pores of the support and hence reduce its overall capacity to grow carbon. As a practical matter, therefore, metal loadings in excess of five layers should be avoided. Metal loadings on the order of 1.5 to 3 are preferred with metal loadings on the order of two layers being especially preferred.

Incidentally, it should be appreciated that in porous particles having the relatively high surface areas reported herein, the amount of surface area attributable to the external surface of the particles is essentially negligible. Accordingly, even if the entire external surfaces of the particles also carry deposited iron, the amount of this deposited iron will be negligible or essentially negligible compared with the amount deposited in the body of the support particles.

The Group VIII metal can be deposited into the pores of the porous support by any of the well known techniques for accomplishing such impregnations. Normally, this will be accomplished by impregnating the porous support with a liquid containing the Group VIII metal or a compound thereof in solution, drying and, if necessary, heat treating in an appropriate atmosphere or series of atmospheres so as to recover the metal in elemental form.

For example, the catalytic material of Example 10 reported below was produced as follows. 46.7 g of $Fe(NO_3)_3 \cdot 9H_2O$ were dissolved in 20 ml of water. This solution was added dropwise with stirring to 31.7 gms. of the hydrothermally treated silica described above having a surface area of 53 $m^2/g$. and a pore volume of 1.22 ml./g. Addition was done gradually and with enough stirring to maintain a homogeneous appearance of the catalyst. The impregnated catalyst was then dried overnight at 110°C. In the early stages of drying, the catalyst was stirred every half hour to break-up a soft dark brown crust which formed on the surface. After drying, the catalyst appeared cinnamon in colour, uniform and free-flowing. The catalyst was then calcined in air at 150°C for 30 minutes and further at 400°C for an additional 6 hours to decompose the iron nitrate and form iron oxide. Next, the catalyst was placed in a 25 mm. inside diameter quartz fluid-bed reactor. After raising the temperature to 550°C under a nitrogen atmosphere, the gas stream was changed to hydrogen and maintained for 90 minutes. The temperature was then increased to 650°C and maintained there for an additional 30 minutes in the hydrogen atmosphere whereby the oxidized iron was reduced to elemental iron. The catalyst was then cooled in an atmosphere of nitrogen and then removed from the reactor once reaching room temperature. At room temperature, the catalyst was stable to atmospheric oxygen.

Other techniques for depositing the Group VIII metal can be employed. For example, organic compounds such as iron carbonyl or phenyl iron dissolved in a suitable organic solvent such as n-pentane could be employed as the impregnating solution. Or inorganic salts of Group VIII metals dissolved in organic solution (e.g. methanol) could be used, as impregnating solution.

The catalytic material used according to the invention as described above has many unique features. It catalyzes the deposition of carbon by disproportionation of carbon monoxide at a high rate with the vast majority of the carbon being deposited within its pores. It also shows high activity in the carbon removal step by formation of methane through contract with hydrogen as also shown in the above-noted Belgian patent. Furthermore, it is able to withstand repeated cycling between carbon deposition and removal without significant loss in activity or without significant attrition even though carbon loadings (weight carbon/total particle weight) as high as 20% and higher are realized during carbon deposition. It is also resistant to temperature changes which may occur because the carbon deposition and carbon removal steps in the carbon cycle occur at different temperatures. Further it is readily fluidized in conventional fluidized bed reactors operating with conventional flow rates such as 0.01 to 10 ft (0.305—305 cm), preferably 0.5 to 5 ft (15.2—125 cm), more normally about 1 to 2 ft (30.5—61 cm)/sec. (gas velocity). Finally, it exhibits little or no growth or carbon fibers on its surface and hence problems due to the fragility of these fibers and agglomeration through comingling of these fibers is avoided. Because of these properties, the catalytic material is ideally suited for carrying out the carbon deposition/removal procedure described in the above-mentioned Belgian patent in a fluidized bed system.

Working Examples

A number of different catalytic materials of use according to the present invention were produced by the preferred technique of impregnating a support (usually the preferred hydrothermally processed silica described above) with a solution of a compound of a Group VIII metal, calcining to convert the metal compound to the corresponding metal oxide and then reducing the oxide to the metal. Usually, the calcination reduction procedure was the same as described above in connection with the preferred embodiment, although in some instances more severe reduction conditions (i.e. higher temperatures) were employed. Also in some instance additional elements such as potassium and chromium were incorporated into the catalyst.

5

The catalytic materials so made were subjected to repeated carbon deposition/carbon removal cycles. Normally, carbon was removed by methanation although sometimes carbon was removed by steaming and in other instances carbon was removed by methanation first followed by steaming in the later cycles. Unless otherwise indicated, in each example, 40 ml. of the catalyst were placed in a 25 mm. inside diameter 90 cm. long fluid-bed reactor equipped with a quartz frit supporting the catalyst located 25 cm. from the bottom. Incoming feed gas streams were dispersed by a coarse gray glass sparger inserted 12 cm. up from the bottom of the quartz tube, and at the top of the tube another gas sparger of fine porosity was inserted 4 cm. from the top to filter catalyst fines from the tailgas. In each experiment, 40 ml. of catalyst was placed in the reactor. Next, the catalyst was brought to 450°C under nitrogen atmosphere. When the temperature stabilized at 450°C, $H_2$ and CO flows were added at 46 ml./min. and 195 ml./min. respectively. Under these carbon deposition conditions, the catalyst became black within about a half-an-hour. The above conditions were maintained for about one hour and then the temperature was raised to 550°C and kept at this temperature for one hour for the first carbon deposition step. Then the carbon monoxide and nitrogen flows were shut off and the $H_2$ flow increased to 129 ml./min. to accomplish methanation. Methanation was carried out for about 2 hours to complete the first carbon deposition/methanation step. Thereafter, the carbon deposition/carbon removal cycle was repeated a number of times under the same conditions except that carbon deposition was carried out at 450°C in the second and subsequent cycles rather than 550°C. All reactions were carried out at atmospheric pressure. In a number of examples carbon removal was accomplished by steaming.

During each carbon deposition and carbon removal step, the gross reaction product was recovered and analyzed. From this analysis, the rate of carbon deposition and rate of carbon removal, both during methanation and steaming, were determined. The identity of the catalyst, including the identity, surface area and pore volume of the support, the overall catalyst composition and average rates of carbon deposition and carbon removal are given in the following Table II. The carbon deposition rate is reported in grams carbon deposited per hour per gram metal on the catalyst and grams carbon removed is similarly reported in grams carbon removed per hour per gram metal of catalyst. During carbon removal by methanation, a slight amount of the carbon occasionally forms carbon dioxide or carbon monoxide. This is excluded from the carbon removal rate. Also, since the first carbon deposition/carbon removal step serves to activate the catalyst, the first cycle is omitted from the reported average rates.

TABLE II

| Example No. | Support | | | Catalyst composition | Steam temp. (°C) | Rates | | | Remarks |
|---|---|---|---|---|---|---|---|---|---|
| | Composition | Surface area m²/g | Pore vol. ml/g | | | Carbon deposit | Carb removal | | |
| | | | | | | | meth. | steam | |
| 1 | $SiO_2$ | 120 | 1.53 | 15.0% Fe 85.0% $SiO_2$ | — | 0.66 | 0.18 | — | |
| 2 | $SiO_2$ | 62 | 1.29 | 15.0 Fe 85.0 $SiO_2$ | — | 0.42 | 0.11 | — | The fines (<325 mesh (0.045 mm)) were removed. |
| 3 | $SiO_2$ | 62 | 1.29 | 15.0% Fe 85.0% $SiO_2$ | — | 0.74 | 0.38 | — | To the catalyst of Ex. 2 (after 325 removed) inert fines (<325) were added. |
| 4 | $SiO_2$ | 61 | 1.46 | 15.0% Fe 85.0% $SiO_2$ | — | 0.66 | 0.27 | — | |
| 5 | $SiO_2$ | 46 | 1.39 | 15.0% Fe 85.0% $SiO_2$ | 550—750 | 0.59 | 0.31 | — | At 750°C good steaming reaction but oxidised catalyst. |
| 6 | $SiO_2$ | * | * | 16.9% Fe 83.1% $SiO_2$ | — | 0.31 | 0.21 | — | Support calcined before metal addition. |
| 7 | $SiO_2$ | 75 | 1.39 | 16.9% Fe 83.1% $SiO_2$ | — | 0.62 | 0.23 | — | |
| 8 | $SiO_2$ | 129 | 1.35 | 16.9% Fe 83.1% $SiO_2$ | — | 0.59 | 0.29 | — | |
| 9 | $SiO_2$ | * | * | 16.9% Fe 83.1% $SiO_2$ | — | 0.50 | 0.20 | — | |
| 10 | $SiO_2$ | 53 | 1.22 | 16.9% Fe 93.1% $SiO_2$ | — | 0.55 | 0.25 | — | |

* Not available.

TABLE II (contd.)

| Example No. | Support Composition | Surface area m²/g | Pore vol. ml/g | Catalyst composition | Steam temp. (°C) | Carbon deposit | Carb removal meth. | Carb removal steam | Remarks |
|---|---|---|---|---|---|---|---|---|---|
| 11 | $SiO_2$ | 58 | 1.39 | 15.0% Ni<br>85.0% $SiO_2$ | — | 0.74 | 0.28 | — | |
| 12 | $SiO_2$ | 58 | 1.39 | 15.0% Ni<br>85.0% $SiO_2$ | 700<br>650<br>600<br>550 | after<br>steam<br>1.31 | | 2.30<br>2.18<br>1.97<br>1.00 | Unexpected increase in deposition rate *after* steaming—20 ml catalyst |
| 13 | $SiO_2$ | 58 | 1.39 | 10.0% Fe<br>90.0% $SiO_2$ | — | 1.03 | 0.42 | — | |
| 14 | $SiO_2$ | 45 | 1.18 | 15.0% Fe<br>85.0% $SiO_2$ | — | 0.55 | 0.26 | — | |
| 15 | $SiO_2$ | | | 15.0% Fe<br>85.0% $SiO_2$ | — | 0.61 | 0.25 | — | |
| A | $SiO_2$ | 688 | 0.95 | 15.0% Fe<br>85.0% $SiO_2$ | — | 0.00 | | — | Less than mono-layer Fe. |
| B | $SiO_2$ | 688 | 0.95 | 30.0% Fe<br>70.0% $SiO_2$ | — | 0.00 | | — | Less than mono-layer Fe. |
| C | $SiO_2$ | 128 | 0.32 | 15.0% Fe<br>85.0% $SiO_2$ | — | 0.36 | Not deter-mined | — | Catalyst broke-up and clumped. |
| D | $SiO_2$ | 688 | 0.95 | 71.0% Fe<br>29.0% $SiO_2$ | — | 0.09 | 0.03 | — | Very poor rates, very bad fluidization—20 ml. catalyst |
| 16 | $SiO_2$ | 64 | 1.29 | 15.0% CO<br>85.0% $SiO_2$ | 600—550 | 0.84 | 0.26 | 0.75<br>0.39 | Catalyst didn't fluidize well |
| 17 | $SiO_2$ | 32 | 1.89 | 15.0% Fe<br>85.0% $SiO_2$ | — | 0.63 | 0.29 | — | |

0 082 222

TABLE II (contd.)

| Example No. | Support Composition | Surface area m²/g | Pore vol. ml/g | Catalyst Composition | Steam temp. (°C) | Carbon deposit | Carb removal meth. | Carb removal steam | Remarks |
|---|---|---|---|---|---|---|---|---|---|
| E | Alundum | 1.0 | 0.57 | 5.0% Fe 95.0% Alundum | — | | | — | Support contained many <50A (5 nm) pores. Catalyst was not homogeneous and could not be used. |
| 18 | SiO₂ | 58 | 1.39 | 15.0% Ni 85.0% SiO₂ 0.5% K | 600—550 | 0.80 | — | 0.53 0.46 | Added K did *not* improve catalyst. |
| 19 | 75 SiO₂/25 Al₂O₃ | 166 | 0.98 | 15.0% Fe 85.0% SiO₂/ Al₂O₃ | — | 0.68 | 0.19 | — | Fe reduced with H₂ at 725°C. Very good fluidization |
| 20 | SiO₂ | 58 | 1.39 | 24.0% Fe 76.0% SiO₂ | — | 0.42 | 0.16 | — | |
| 21 | SiO₂ | 245 | 1.41 | 15.0% Fe 85.0% SiO₂ | — | 0.65 | 0.23 | — | Activated at 700°C in H₂ |
| 22 | SiO₂ | 58 | 1.39 | 10.0% Ni 5.0% Cr 85.0% SiO₂ | 550 | 1.07 | — | 0.91 | Reported rates based on Ni content only. |
| 23 | Al₂O₃ | 61 | 0.45 | 15.0% Ni 85.0% Al₂O₃ | — | 0.45 | 0.15 | — | |
| 24 | SiO₂ | 6 | 1.24 | 15.0% Fe 85.0% SiO₂ | — | 0.58 | 0.27 | — | |
| 25 | Al₂O₃ | 195 | 0.58 | 15.0% Ni 85.0% Al₂O₃ | — | 0.33 | 0.10 | — | |
| 26 | SiO₂ | 42 | 2.45 | 15.0% Ni 85.0% SiO₂ | — | 1.31 | 0.49 | — | Catalyst broke apart because of overloading with carbon during carbon deposition. |

From the above, it can be seen that catalyst used in accordance with the invention exhibit good rates for carbon deposition and carbon removal. In addition from Comparative Examples A, B and D it can be seen that catalysts made from supports having surface areas which are too high exhibit essentially no activity. Furthermore, from Comparative Example C it can be seen that catalyst made with a support having an acceptable surface area but a pore volume which is too small also are unsuitable. In addition, from Comparative Example E it can be seen that catalyst made from a support having a surface area too low are also ineffective. Thus, it will be appreciated that only by selecting the support to have the properties indicated is it possible to form a dimensionally stable catalyst exhibiting good activity in carbon deposition and removal and which further is suitable for use as a fluid-bed catalyst.

**Claims**

1. A fluid-bed process for converting carbon monoxide to a high energy gas comprising contacting a particulate, free-flowing, fluid-bed catalyst comprising a porous catalyst support having a surface area of greater than 1 to less than 350 $m^2/g$ and a pore volume of 0.4 to 3.0 ml/g and a Group VIII metal or mixture thereof in elemental form on the pore surface of said support, said Group VIII metal or mixture being present in an amount not exceeding that equivalent to at least five atomic layers with an upflowing gas stream containing carbon monoxide so as to fluidize said catalytic material and deposit carbon in the pores of said catalytic material and thereafter contacting said catalyst with a carbon removal gas selected from hydrogen and steam so as to fluidize said catalytic material and remove carbon from said catalytic material.

2. A process as claimed in claim 1 characterised in that the Group VIII metal or mixture is present in an amount equivalent to at least one atomic layer.

3. A process as claimed in claim 1 or claim 2 characterised in that the catalyst has a particle size distribution such that 80% of said catalyst is between 5 to 300 micrometers in size and a density such that said catalyst will form a fluidized bed when contacted with an upflowing gas flowing at a velocity of 0.01 to 10 ft (0.305—305 cm)/sec.

4. A process as claimed in any of claims 1 to 3 characterised in that the surface area of the catalyst is 6 to 250 $m^2/g$ and said pore volume is 1 to 2 ml/g.

5. A process as claimed in any of claims 1 to 4 characterised in that the support exhibits no measurable pores having a pore diameter of less than 50 angstroms (5 nm) as measured by the mercury porousimeter technique.

6. A process as claimed in any of claims 1 to 5 characterised in that said support is silica.

7. A process as claimed in any of claims 1 to 6 characterised in that the Group VIII metal is iron, cobalt or nickel.

8. A process as claimed in any of claims 1 to 7 characterised in that the gas flow rates of said carbon monoxide during carbon deposition and said carbon removal gas during carbon removal are 0.01 to 10 ft (0.305 to 305 cm)/sec.

9. A process as claimed in any of claims 1 to 8 characterised in that said carbon removal gas is hydrogen.

10. A process as claimed in any of claims 1 to 8 characterised in that the carbon removal gas is steam.

**Patentansprüche**

1. Wirbelschichtbettverfahren zum Umwandeln von Kohlenmonoxid in ein energiereiches Gas durch Kontakt eines teilchenförmigen freifließenden Wirbelschicht-Katalysators, der einen porösen Katalysatorträger, mit einer spezifischen Oberfläche von größer als 1 bis weniger als 350 $m^2/g$ und ein Porenvolumen von 0,4 bis 3,0 ml/g hat une ein Metall der Gruppe VIII oder ein Gemisch davon in Elementarform an der Porenoberfläche von dem Träger enthält, wobei das Metall der Gruppe VIII oder das Gemisch in einer Menge vorliegt, die die zumindest fünf Atomschichten äquivalente nicht überschreitet, mit einem aufwärtsströmenden Gasstrom der Kohlenmonoxid enthält, um das katalytische Material aufzuwirbeln und in den Poren des katalytischen Materials Kohlenstoff abzulagern und anschließenden Kontakt des Katalysators mit einem kohlenstoffentfernenden Gas, ausgewählt aus Wasserstoff und Dampf, um das katalytische Material aufzuwirbeln und Kohlenstoff vom katalytischen Material zu entfernen.

2. Verfahren nach Anspruch 1, dadurch gekennzeichnet, daß das Metall der Gruppe VIII oder das Gemisch in einer Menge vorliegen, äquivalent zu mindestens einer Atomschicht.

3. Verfahren nach Anspruch 1 oder 2, dadurch gekennzeichnet, daß der Katalysator eine Teilchengrößenverteilung solcher Art hat, daß 80 % des Katalysators zwischen 5 bis 300 micrometer Größe liegen und eine derartige Dichte, daß der Katalysator ein Wirbelstrombett bildet, wenn er mit einem aufwärts strömenden Gas mit einer Geschwindigkeit von 0,01 bis 10 ft (0,306 bis 305 cm)/sec. in Kontakt gebracht wird.

4. Verfahren nach einem der Ansprüche 1 bis 3, dadurch gekennzeichnet, daß die spezifische Oberfläche des Katalysators 6 bis 250 $m^2/g$ und das Porenvolumen 1 bis 2 ml/g ist.

5. Verfahren nach einem der Ansprüche 1 bis 4, dadurch gekennzeichnet, daß der Träger keine meßbaren Poren, mit einem Porendurchmesser von weniger als 50 Angström (5 nm), gemessen durch die Quecksilber-Porosimeter-Technik, zeigt.

6. Verfahren nach einem der Ansprüche 1 bis 5, dadurch gekennzeichnet, daß der Träger Siliziumdioxid ist.

7. Verfahren nach einem der Ansprüche 1 bis 6, dadurch gekennzeichnet, daß das Metall der Gruppe VIII Eisen, Kobalt oder Nickel ist.

8. Verfahren nach einem der Ansprüche 1 bis 7, dadurch gekennzeichnet, daß die Gasströmungsgeschwindigkeiten des Kohlenmonoxides, während der Kohlenstoffablagerung und des Gases zur Entfernung des Kohlenstoffes, während der Kohlenstoffentfernung 0,01 bis 10 ft (0,305 bis 305 cm)/sec. sind.

9. Verfahren nach einem der Ansprüche 1 bis 8, dadurch gekennzeichnet, daß das Gas zur Kohlenstoffentfernung Wasserstoff ist.

10. Verfahren nach einem der Ansprüche 1 bis 8, dadurch gekennzeichnet, daß das Gas zur Kohlenstoffentfernung Dampf ist.

## Revendications

1. Procédé à lit fluidisé pour la conversion de l'oxyde de carbone en un gaz à énergie élevée, consistant à mettre en contact avec un courant de gaz, contenant de l'oxyde carbone et en écoulement ascendant, un catalyseur à lit fluidisé, à l'état subdivisé et s'écoulant librement, comprenant un support poreux de catalyseur offrant une surface spécifique supérieure à 1 et inférieure à 350 m$^2$/g et un volume de pores de 0,4 à 3,0 ml/g et l'un des métaux du Groupe VIII ou un mélange de ceux-ci, sous forme élémentaire à la surface des pores dudit support, ledit métal ou mélange du Groupe VIII étant présent dans une quantité n'excédant pas celle équivalente à au moins cinq couches atomiques, de manière à fluidiser ladite matière catalytique et à déposer du carbone dans les pores de ladite matière catalytique, puis à mettre ensuite ledit catalyseur en contact avec un gaz d'extraction de carbone choisi parmi l'hydrogène et la vapeur d'eau de manière à fluidiser ladite matière catalytique et à extraire de cette matière catalytique le carbone.

2. Procédé tel que revendiqué dans la revendication 1, caractérisé en ce que le métal ou mélange du Groupe VIII est présent dans une quantité équivalente à au moins une couche atomique.

3. Procédé tel que revendiqué dans la revendication 1 ou la revendication 2, caractérisé en ce que le catalyseur présente une répartition granulométrique telle que 80% dudit catalyseur sont d'une taille comprise entre 5 et 300 microns et une densité telle que ledit catalyseur forme un lit fluidisé lorsqu'on le met en contact avec un écoulement de gaz dirigé vers le haut sous une vitesse de 0,01 à 10 pieds (0,305—305 cm) par seconde.

4. Procédé tel que revendiqué dans l'une quelconque des revendications 1 à 3, caractérisé en ce que la surface spécifique du catalyseur est de 6 à 250 m$^2$/g et ledit volume de pores est de 1 à 2 ml/g.

5. Procédé tel que revendiqué dans l'une quelconque des revendications 1 à 4, caractérisé en ce que le support n'offre pas de pores mesurables ayant un diamètre de pore inférieur à 50 angströms (5 nm), la mesure étant effectuée à l'aide de la technique du porosimètre au mercure.

6. Procédé tel que revendiqué dans l'une quelconque des revendications 1 à 5, caractérisé en ce que le dit support est de la silice.

7. Procédé tel que revendiqué dans l'une quelconque des revendications 1 à 6, caractérisé en ce que le métal du Groupe VIII est le fer, le cobalt ou le nickel.

8. Procédé tel que revendiqué dans l'une quelconque des revendications 1 à 7, caractérisé en ce que les débits de gaz dudit oxyde de carbone au cours du dépôt du carbone, et dudit gaz d'extraction du carbone au cours de l'extraction du carbone sont de 0,01 à 10 pieds (0,305 à 305 cm) par seconde.

9. Procédé tel que revendiqué dans l'une quelconque des revendications 1 à 8, caractérisé en ce que ledit gaz d'extraction du carbone est de l'hydrogène.

10. Procédé tel que revendiqué dans l'une quelconque des revendications 1 à 8, caractérisé en ce que le gaz d'extraction du carbone est de la vapeur d'eau.